# EUROPÄISCHE PATENTANMELDUNG

(11) **EP 4 151 727 A1**
(43) Veröffentlichungstag der Anmeldung: **22.03.2023**
(21) Anmeldenummer: 22184632.2
(22) Anmeldetag: 21.06.2018
(51) Int. Cl.: C12N 9/80, C12N 9/78, C12P 13/02, C12P 7/18, C12P 7/44, C12P 13/00

(54) **NEUE URETHANASEN FÜR DEN ENZYMATISCHEN ABBAU VON POLYURETHANEN**

(62) Teilanmeldung aus: 18179032.0
(71) Anmelder: Covestro Deutschland AG, 51373 Leverkusen (DE)
(72) Erfinder: Bittner, Natalie, 50226 Frechen (DE); Nefzger, Hartmut, 50259 Pulheim (DE); Behnken, Gesa, 50670 Köln (DE); Jaeger, Gernot, 50733 Köln (DE); Behnken, Swantje, Sacramento, CA 95864 (US); Reisky, Lukas, 50996 Köln (DE)
(74) Vertreter: Levpat

(57) **Zusammenfassung**

Die vorliegende Erfindung betrifft eine neue Urethanase für den enzymatischen Abbau von Polyurethanen sowie ein enzymatisches Verfahren für den vollständigen Abbau von Polyurethanen in definierte Monomere.

## Beschreibung

Die vorliegende Erfindung betrifft neue Urethanasen für den enzymatischen Abbau von Polyurethanen sowie ein enzymatisches Verfahren für den vollständigen Abbau von Polyurethanen in definierte Monomere.

Polyurethane sind in vielen Bereichen des normalen Lebens etabliert. Sie finden sich zum Beispiel in Weichschäumen (Matratzen, Schwämme, Polstermöbel), Hartschäumen (Isolationsmaterialien, Baustoffe), Thermoplasten (Sportschuhe) oder Beschichtungen (Lacke, Farben, Klebstoffe). Aufgrund der stetig steigenden Nachfrage nach den Produkten werden zunehmend größere Mengen produziert. Parallel dazu wächst das Bedürfnis nach Methoden für möglichst nachhaltiges Recycling von nicht mehr benötigten Polyurethanprodukten, die eine Wiederverwertung von Bausteinen der Polymere erlaubt. Dafür müssen die Bindungen in den Polyurethanen gezielt gespalten werden, um definierte Abbauprodukte erhalten zu können und diese damit wiederverwertbar zu machen.

Neben den physiologischen Funktionen, welche Enzyme in lebenden Organismen erfüllen, können sie auf vielfältige Weise zur Katalyse chemischer Reaktionen außerhalb dieses Kontextes genutzt werden. Hierbei können im Vergleich zu konventionell chemischen Prozessen die Reaktionen bei milderen Bedingungen wie niedrigerer Temperatur, neutralem pH-Wert und ohne die Verwendung aggressiver Chemikalien durchgeführt werden. Dadurch kann Energie gespart, die Bildung von Nebenprodukten minimiert und die Umwelt geschont werden, was zur Reduktion der Betriebskosten beiträgt. Durch den Einsatz von Enzymen wird es teilweise erst ermöglicht, dass labile Edukte umgesetzt werden können (Jaeger, K.-E. & Reetz, M. T. (1998) Microbial lipases form versatile tools for biotechnology. Trends in biotechnology, 16, 396-403). Weiterhin sind Enzyme häufig regio-, stereo- und enantioselektiv, was die Aufreinigung der Produkte deutlich erleichtert und damit die effiziente Synthese schwer zugänglicher Produkte ermöglichen kann (Hasan, F., Shah, A. A. & Hameed, A. (2006) Industrial applications of microbial lipases. Enzyme and Microbial Technology, 39, 235-251).

Das Recycling von Polyurethanen wird hauptsächlich durch thermisches Recycling durchgeführt. Im Allgemeinen läuft dieser Prozess bei sehr hohen Temperaturen und mit sehr langen Reaktionszeiten im Batchprozess, sowie unter Einsatz von Katalysatoren ab. Dabei kann es passieren, dass ein thermischer Abbau der Polymerketten in Crackreaktionen zu unerwünschten und undefinierten Abbauprodukten oder auch die Bildung von Epoxidringen geschieht, welche zu einer hohen Geruchsbelastung und nachteiligen Vernetzungen der Ketten im recyclierten Rohstoff führt und damit insbesondere eine personennahe Wiederverwendung, insbesondere bei der Schaumstoffproduktion für den Möbel- und Matratzenbauunmöglich machen kann. Alternativ wird auch eine vollständige Verbrennung und damit energetische Verwertung durchgeführt, dabei wird zwar Energie gewonnen aber eine effiziente Wiederverwendung der Polymerbausteine entfällt.

Es ist bekannt, dass Polyurethane in gewissem Maße von Bakterien und Pilzen abgebaut werden können. Dabei sind Polyesterpolyurethane deutlich anfälliger gegenüber mikrobiellem bzw. enzymatischem Abbau als Polyetherpolyurethane (Nakajima-Kambe, T., Shigeno-Akutsu, Y., Nomura, N., Onuma, F. & Nakahara, T. (1999) Microbial degradation of polyurethane, polyester polyurethanes and polyether polyurethanes. Applied microbiology and biotechnology, 51, 134-140).

Der Abbau von Polyesterpolyurethanen lässt sich leicht über die Hydrolyse der Esterbindungen realisieren. Der relativ einfache Abbau von Polyestern ist nicht weiter verwunderlich, da Esterbindungen in hydrophoben Substraten in der Natur auch beim Abbau von Lipiden gespalten werden müssen und auch Polyester ohne Urethanbindungen relativ leicht durch Esterasen und Lipasen abgebaut werden können (Marten, E., Müller, R.-J. & Deckwer, W.-D. (2003) Studies on the enzymatic hydrolysis of polyesters I. Low molecular mass model esters and aliphatic polyesters. Polymer degradation and stability, 80, 485-501; Marten, E., Müller, R.-J. & Deckwer, W.-D. (2005) Studies on the enzymatic hydrolysis of polyesters. II. Aliphatic-aromatic copolyesters. Polymer degradation and stability, 88, 371-381.). In verschiedenen Literaturstellen wurden zum Abbau von Polyurethan verwendete Enyzme als Esterasen charakterisiert (Allen, A. B., Hilliard, N. P. & Howard, G. T. (1999) Purification and characterization of a soluble polyurethane degrading enzyme from Comamonas acidovorans. International biodeterioration & biodegradation, 43, 37-41; Blake, R., Norton, W. & Howard, G. (1998) Adherence and growth of a Bacillus species on an insoluble polyester polyurethane. International biodeterioration & biodegradation, 42, 63-73; Crabbe, J. R., Campbell, J. R., Thompson, L., Walz, S. L. & Schultz, W. W. (1994) Biodegradation of a colloidal esterbased polyurethane by soil fungi. International biodeterioration & biodegradation, 33, 103-113; Darby, R. T. & Kaplan, A. M. (1968) Fungal susceptibility of polyurethanes. Applied microbiology, 16, 900-905; Howard, G. T., Norton, W. N. & Burks, T. (2012) Growth of Acinetobacter gerneri P7 on polyurethane and the purification and characterization of a polyurethanase enzyme. Biodegradation, 23, 561-573; Kaplan, A. M., Darby, R. T., Greenberger, M. & Rodgers, M. (1968) Microbial deterioration of polyurethane systems. Dev Ind Microbiol, 82, 362-371; Kay, M., Morton, L. & Prince, E. (1991) Bacterial degradation of polyester polyurethane. International biodeterioration, 27, 205-222; Vega, R. E., Main, T. & Howard, G. T. (1999) Cloning and expression in Escherichia coli of a polyurethane-degrading enzyme from Pseudomonas fluorescens. International biodeterioration & biodegradation, 43, 49-55). Dort gibt es keinen eindeutigen Nachweis auf die Spaltung der Urethanbindung, da die Enzymcharakterisierung in keinem Fall auf Grundlage der Spaltung eines Moleküls mit Urethangruppe durchgeführt wurde.

In vielen Publikationen und Patenten ist der Abbau von Poly(ester)urethanen mit Pilzen oder Bakterien beschrieben. Jedoch zielt der Abbau meist nur auf die relativ leicht zu spaltenden Esterbindungen und wird meist nur durch eine makroskopische Beobachtung des Polymerabbaus belegt. Es gibt hier keinen gesteuerten Abbau von Ester- und Urethanbindungen, wie in der vorliegenden Erfindung, und es ergeben sich häufig lange Abbauzeiten. Diese Veröffentlichungen zeigen, dass Urethanasen weit verbreitete Enzyme sind, jedoch belegen sie nicht deren spezifischen Fähigkeiten, Einsatzmöglichkeiten und Gruppenzugehörigkeit, wie in der vorliegenden Erfindung genutzt. (JP09192633, Tang, Y. W., Labow, R. S., Santerre, J. P. (2003) Enzyme induced biodegradation of polycarbonate-polyurethanes: dose dependence effect of cholesterol esterase. Biomaterials 24 (12), 2003-2011, Vega, R. E., Main, T. & Howard, G. T. (1999) Cloning and expression in Escherichia coli of a polyurethane-degrading enzyme from Pseudomonas fluorescens. International biodeterioration & biodegradation, 43, 49-55)

Zum enzymatischen Abbau von Poly(ester)urethanen ist ein Abbauverfahren bekannt, bei dem im ersten Schritt aus einer Kultivierung von *Comamonas acidovorans* Stämmen eine Esterase gewonnen wird, indem als Kohlenstoffquelle nur Poly(ester)urethan zur Verfügung steht. In einem aufwendigen Aufarbeitungsschritt wird die Esterase abgetrennt und für den Abbau von Poly(ester)urethanen im Batch eingesetzt. Hierbei ergeben sich lange Abbauzeiten in einem mehrstufigen Verfahren und keine Belege für eine spezifische Spaltung der Urethanbindungen. (JP 09201192, JP 10271994)

In verschiedenen Patenten und Publikationen wird der Abbau von Poly(ester)urethanen mit Cutinasen, Esterasen und/oder Lipasen beschrieben. Jedoch zielt der Abbau hier nur auf die relativ einfache Spaltung der Esterbindungen, nicht aber spezifisch auf die Urethanbindungen. Außerdem wird keine gezielte Kombination von Ester- und Urethanbindungen spaltenden Enzymen zur gezielten Steuerung des Abbaus beschrieben. Es ist davon auszugehen, dass die beschriebenen Verfahren nur zu einer geringen oder keiner Spaltung der Urethanbindung führen. Damit können eingesetzte Diamine nicht effizient wiedergewonnen werden. (EP 0968300, US 6,180,381)

In WO 2013/134801 wird der Abbau von aromatischen Polyurethanen, basierend auf Polyetherpolyolen, unter Anwendung eines Enzymes der Klasse EC 3 beschrieben. Dabei sind keine spezifischen Enzymsequenzen angegeben, so dass die Spezifität des Verfahrens in dem Abbau bestimmter Urethanbindungen, sowie der kontrollierten Spaltung von Ester- und gesondert der Spaltung von Urethanbindungen, wie in der vorliegenden Erfindung gezeigt, in dem genannten Patent nicht belegt ist. Weiterhin gibt es keine Beschreibung einer Regelung des pH Wertes der Mischung während des Polymerabbaus, zur Erhaltung der Urethanaseaktivität. Weiterhin ist kein regioselektiver Abbau beschrieben, sowie kein Abbau von aliphatischen Poly(ester)urethanen.

In WO 2006/019095 wird eine Urethanase und durch Protein Engineering gewonnene Varianten dieses Enzyms beschrieben. Das Enzym kann auf TDA oder MDA beruhende Urethanoligomere spalten. Jedoch werden hier weder Bindungen regioselektiv gespalten noch gibt es eine Anwendung in Kombination mit Esterasen zum Abbau von Polymeren. Zudem sind keine weiteren Urethanasen aus der GatA- oder Aes-Familie, oder irgendeiner anderen Gruppe beschrieben.

Der vorliegenden Erfindung lag somit die Aufgabe zugrunde, weitere Enzyme bereitzustellen, die für die enzymatische Spaltung von Urethanbindungen und vorzugsweise für die Verwendung beim vollständigen enzymatischen Abbau von Polyurethanen einsetzbar sind. Weiterhin sollte ein enzymatisches Verfahren bereitgestellt werden, das den Abbau von Polyurethanen in definierte Monomere ermöglicht.

Diese Aufgabe wird durch die in den Patentansprüchen und in der untenstehenden Beschreibung offenbarten Ausführungsformen gelöst.

In einer ersten Ausführungsform betrifft die vorliegende Erfindung ein Polypeptid mit einer Aminosäuresequenz ausgewählt aus der Gruppe bestehend aus SEQ ID Nr. 3, SEQ ID Nr. 2, SEQ ID Nr. 4, SEQ ID Nr. 5, SEQ ID Nr. 6, SEQ ID Nr. 8, SEQ ID Nr. 10 und Varianten dieser Polypeptide oder ein Polypeptid mit einer Aminosäuresequenz gemäß SEQ ID Nr. 7 oder eine Variante davon, dadurch gekennzeichnet dass das Polypeptid Urethanaseaktivität aufweist.

### Referenz für die erwähnten Polypeptide

| **SEQ ID Nr.** | **Interne Bezeichnung** | **Bezeichnung in Studie** |
|---|---|---|
| 1 | Enz01 | GatA61 |
| 2 | Enz02 | Aes70 |
| 3 | Enz03 | Aes72 |
| 4 | Enz04 | Aes170 |
| 5 | Enz05 | Aes174 |
| 6 | Enz06 | Aes175 |
| 7 | Enz07 | GatA197 |
| 8 | Enz08 | Aes214 |
| 9 | Enz09 | GatA250 |
| 10 | Enz10 | AesGö56 |
| 11 | Ref01 | SB12 |
| 12 | Ref02 | SB23 |

### Polypeptid

Der Begriff "Polypeptid" ist dem Fachmann gut bekannt. Er bezeichnet eine Kette von wenigstens 50, vorzugsweise wenigstens 70 Aminosäuren, die durch Peptidbindungen miteinander verknüpft sind. Ein Polypeptid kann sowohl natürlich vorkommende als auch synthetische Aminosäuren enthalten. Vorzugsweise enthält es die bekannten proteinogenen Aminosäuren.

Für SEQ ID Nr. 1 bis 5, 9 und 10 wird eine Variante durch Hinzufügung, Deletion oder Austausch von bis zu 10 %, bevorzugt bis zu 5 %, der im jeweiligen Polypeptid enthaltenen Aminosäuren erhalten. Eine bevorzugte Variante von SEQ ID Nr. 7 wird durch Hinzufügung, Deletion oder Austausch von bis zu 5 %, der in SEQ ID Nr. 7 definierten Aminosäuren erhalten. Besonders bevorzugte Varianten der vorgenannten Polypeptide werden durch Hinzufügung, Deletion oder Austausch von bis zu 20, bevorzugt bis zu 10 und noch stärker bevorzugt bis zu 5 Aminosäuren der offenbarten Sequenzen erhalten. Bevorzugte Varianten von SEQ ID Nr. 6 und SEQ ID Nr. 8 werden durch Hinzufügung, Deletion oder Austausch von bis zu 3, stärker bevorzugt bis zu 2 Aminosäuren erhalten. Grundsätzlich können die vorgenannten Modifikationen an jeder beliebigen Stelle des Polypeptids kontinuierlich oder diskontinuierlich erfolgen. Bevorzugt erfolgen sie aber nur am N-Terminus und/oder am C-Terminus des Polypeptids. Jede erfindungsgemäße durch Hinzufügung, Austausch oder Deletion von Aminosäuren erhaltene Variante ist aber durch Urethanaseaktivität wie weiter unten in dieser Anmeldung definiert gekennzeichnet.

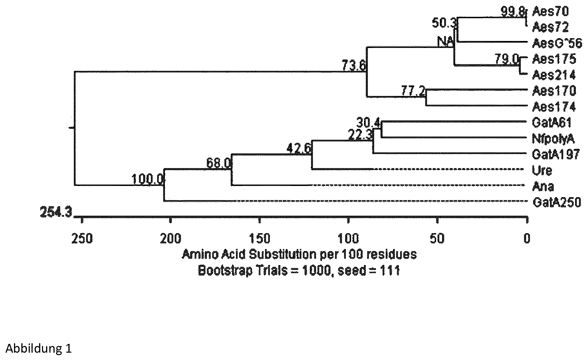

Die Polypeptide, wie sie durch SEQ ID Nr. 3, SEQ ID Nr. 2, SEQ ID Nr. 4, SEQ ID Nr. 5, SEQ ID Nr. 6, SEQ ID Nr. 8 und SEQ ID Nr. 10 definiert sind, bilden eine von dem bisher einzigen bekannten Enzym mit Urethanaseaktivität, Ure, phylogenetisch unterschiedene Gruppe (siehe Abbildung 1). Aus dieser Gruppe waren bisher noch keine Enzyme mit entsprechender Aktivität bekannt. Im Folgenden wird diese Gruppe von Polypeptiden auch als "Aes-ähnlich" bezeichnet.

### Urethanaseaktivität

Der Begriff "Urethanaseaktivität" bezieht sich auf die Fähigkeit eines Polypeptids, die Spaltung einer Urethangruppe enzymatisch zu katalysieren. Pro Mol Urethangruppe entsteht hierbei ein Mol Amin, ein Mol Alkohol und ein Mol CO₂.

Bei der Urethangruppe kann es sich um eine aromatisch oder eine aliphatisch gebundene Urethangruppe handeln. Bei einer aromatisch gebundenen Urethangruppe ist das Stickstoffatom direkt an einen aromatischen Ring gebunden. Bei einer aliphatisch gebundenen Urethangruppe ist das Stickstoffatom an einen Alkylrest gebunden. Vorzugsweise handelt es sich um einen unverzweigten Alkylrest mit wenigstens einem, stärker bevorzugt wenigstens zwei und am stärksten bevorzugt wenigstens drei Kohlenstoffatomen. In einer bevorzugten Ausführungsform der vorliegenden Erfindung ist das Polypeptid mit Urethanaseaktivität in der Lage eine aromatisch gebundene Urethangruppe enzymatisch zu spalten.

Ob ein Polypeptid eine Urethanaseaktivität besitzt, kann durch die Spaltung geeigneter Modellsubstrate überprüft werden.

Für die Fähigkeit zur Spaltung aromatisch gebundener Urethangruppen dient vorzugsweise Ethyl-4-Nitrophenylcarbamat (ENPC) als Modellsubstrat. Die Spaltung wird durch die Bestimmung des Konzentrationsanstiegs von 4-Nitroanilin nachgewiesen. Dies erfolgt vorzugsweise photometrisch bei einer Wellenlänge von 405 nm. Die Enzymaktivität wird vorzugsweise in einem Rektionspuffer mit 100 mM K₂HPO_{4/}KH₂PO₄, pH 7 mit 6,25 Vol.-% Ethanol in Anwesenheit von 0,2 mg/L ENPC als Substrat bestimmt. Die Inkubation des Enzyms im Reaktionspuffer mit ENPC erfolgt vorzugsweise bei Raumtemperatur und vorzugsweise für 24 Stunden.

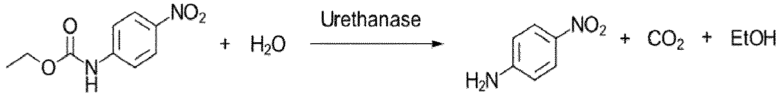

Für die Fähigkeit zur Spaltung aliphatisch gebundener Urethangruppen dient vorzugsweise Ethylphenethylcarbamat (EPEC) als Modellsubstrat. Die Spaltung wird durch die Bestimmung des Konzentrationsanstiegs von Phenethylamin nachgewiesen. Dies erfolgt vorzugsweise durch HPLC. Der verwendete Reaktionspuffer und die Reaktionsbedingungen entsprechen vorzugsweise den oben für ENPC beschriebenen Parametern.

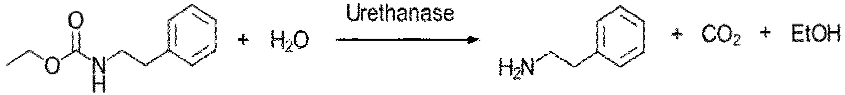

### Enzymatische Spaltung

Der Begriff "enzymatische Spaltung einer Urethangruppe" kennzeichnet, dass die oben beschriebene Spaltung einer Urethangruppe in Anwesenheit eines Polypetids mit Urethanaseaktivität schneller abläuft als bei Inkubation mit dem Reaktionspuffer ohne Enzym unter denselben Reaktionsbedingungen oder bei Inkubation mit dem Reaktionspuffer unter denselben Bedingungen in Anwesenheit eines inaktiven Polypeptids. Als Modell für ein inaktives Polypeptid ist bovines Serumalbumin bevorzugt. Wenn in Anwesenheit eines zu testenden Polypeptids die Spaltung der Urethangruppe schneller abläuft als in einer ansonsten identischen Kontrolle mit BSA, besitzt besagtes Polypeptid Urethanaseaktivität wie in dieser Anmeldung verstanden.

### Verwendung

In einer weiteren Ausführungsform betrifft die vorliegende Erfindung die Verwendung eines Polypeptids mit einer Aminosäuresequenz ausgewählt aus der Gruppe bestehend aus SEQ ID Nr. 3, SEQ ID Nr. 2, SEQ ID Nr. 4, SEQ ID Nr. 5, SEQ ID Nr. 6, SEQ ID Nr. 8, SEQ ID Nr. 10 und Varianten dieser Polypeptide oder eines GatA-ähnlichen Polypeptid mit einer Aminosäuresequenz gemäß SEQ ID Nr. 7 oder einer Variante davon, dadurch gekennzeichnet dass das Polypeptid Urethanaseaktivität aufweist, zur enzymatischen Spaltung von Urethanbindungen.

Alle oben gegebenen Definitionen gelten - soweit nicht explizit anders definiert - auch für diese Ausführungsform.

### Abbau von Urethanen in niedermolekulare Abbauprodukte

In einer weiteren Ausführungsform betrifft die vorliegende Erfindung ein Verfahren zum Abbau von Polyester-Polyurethanen in niedermolekulare Abbauprodukte enthaltend die Schritte
a) Spaltung der im Polyester-Polyurethan enthaltenen Estergruppen; und
b) Behandlung des Polyurethans mit einem Polypeptid, welches Urethanaseaktivität aufweist und eine Aminosäuresequenz ausgewählt aus der Gruppe bestehend aus SEQ ID Nr. 1 bis SEQ ID Nr. 10 und Aminosäuresequenzen mit wenigstens 90 % Sequenzidentität zu den vorgenannten Sequenzen hat;
mit der Maßgabe, dass die Verfahrensschritte a) und b) in beliebiger Reihenfolge oder auch gleichzeitig durchgeführt werden können.

Vorzugsweise wird der Verfahrensschritt a) vor dem Verfahrensschritt b) durchgeführt.

Verfahrensschritt a) wird vorzugsweise mit einer Lipase durchgeführt. Stärker bevorzugt wird eine Lipase verwendet, die in der Lage ist, Tributyrin zu spalten. Noch stärker bevorzugt wird ein Polypeptid verwendet, das eine Aminosäuresequenz wie in SEQ ID Nr. 11 oder SEQ ID Nr. 12 definiert aufweist oder dessen Aminosäuresequenz wenigstens 90 %, bevorzugt wenigstens 95 %, Sequenzidentität mit einer der beiden vorgenannten Sequenzen aufweist und das in der Lage ist Tributyrin zu spalten. Verfahrensschritt a) wird vorzugsweise unter Reaktionsbedingungen durchgeführt, bei denen die verwendete Lipase Aktivität zeigt. Solche Bedingungen können durch Routineexperimente mit gängigen biochemischen Methoden bestimmt werden.

Da die erfindungsgemäßen Polypeptide mit Urethanaseaktivität ihre maximale Aktivität im neutralen Bereich haben, wird der Verfahrensschritt b) vorzugsweise bei einem pH-Wert zwischen 6,0 und 8,0 durchgeführt. Die Einstellung des pH-Wertes kann durch alle dem Fachmann bekannten und geeigneten Basen erfolgen.

Der Begriff "Polyester-Polyurethan" bezeichnet ein Polyurethan, das aus einem oder mehreren Polyesterpolyolen und einem oder mehreren Isocyanaten aufgebaut ist. Das Polyurethan kann geschäumt oder nicht geschäumt vorliegen. Bevorzugt liegt es geschäumt vor. Um die spezifische Oberfläche zu vergrößern, ist es bevorzugt, das Polyurethan vor Durchführung der Verfahrensschritte a) und b) zu zerkleinern. Dies ist insbesondere bevorzugt, wenn Polyurethan in nicht geschäumter Form eingesetzt werden soll. Das Zerkleinern kann auf alle dem Fachmann geläufigen Arten geschehen, vorzugsweise durch Mahlen, Hobeln, Reißen und Schneiden.

Das Polyurethan enthält als Isocyanatkomponente wenigstens ein aromatisches, aliphatisches oder cycloaliphatisches Isocyanat. Bevorzugt enthält das Polyurethan nur aromatische Isocyanate. Bevorzugte aromatische Isocyanate sind Methylendiphenylisocyanat (MDI), Varianten des MDI mit drei und mehr Kernen und Toluylendiisocyanat. Varianten des MDI mit drei und mehr Kernen sind Nebenprodukte der Synthese und können auch in Polyurethanen enthalten sein. Besonders bevorzugt enthält das abzubauende Polyurethan 2,4-Toluylendiisocyanat und 2,6-Toluylendiisocyanat.

Der Begriff "Polyesterpolyol" ist dem Fachmann bekannt, es handelt sich um Polyester, die im Durchschnitt pro Molekül wenigstens 1,5, bevorzugt wenigstens 1,8 und stärker bevorzugt wenigstens 2,0 Hydroxylgruppen enthalten. Besonders bevorzugt weisen die in dem abzubauenden Polyurethan enthaltenen Polyesterpolyole Funktionalität zwischen 1,5 und 6,0 auf. Sie enthalten als Aufbaukomponenten aromatische und/oder aliphatische Polyole sowie aromatische und/oder aliphatischen Polycarbonsäuren in beliebiger Kombination.

Die niedermolekularen Abbauprodukte der polyesterbasierten Polyurethanschäume weisen vorzugsweise ein Molekulargewicht von höchstens 1.000 g/mol auf. Es handelt sich vorzugsweise um
(i) Amine, die von den bei der Herstellung des betreffenden Polyurethans eingesetzten Isocyanaten abgeleitet sind, Beispielsweise im Falle von 2,4-Toluylendiisocyanat um das 2,4-Toluylendiamin; und
(ii) Alkohole und Carbonsäuren, die zum Aufbau der für die Synthese des betreffenden Polyurethans eingesetzten Polyesterpolyole eingesetzt wurden.

Unter einem "Polyol" wird in dieser Anmeldung jede Verbindung mit wenigstens zwei Hydroxylgruppen verstanden. Besagtes Polyol hat vorzugsweise ein Molekulargewicht von höchstens 300 g/mol. Bevorzugte Polyole, die niedermolekulare Abbauprodukte der polyesterbasierten Polyurethanschäume sind, sind ausgewählt aus der Gruppe bestehend aus Ethylengylcol, Diethylenglykol, Triethylenglycol, Propylenglycol, 1,2- Di-Propylenglycol, Neopentylglykol, Glycerin, 1,1,1-Trimethylolpropan, Saccharose, Sorbitol und Pentaerythrit.

Unter einer "Polycarbonsäure" wird in dieser Anmeldung jede Verbindung verstanden, die wenigstens zwei Carboxylgruppen enthält. Besagte Polycarbonsäure hat vorzugsweise ein Molekulargewicht von höchstens 300 g/mol. Bevorzugte Polycarbonsäuren, die niedermolekulare Abbauprodukte der polyesterbasierten Polyurethanschäume sind, sind ausgewählt aus der Gruppe bestehend aus Bernsteinsäure, Glutarsäure, Adipinsäure, Phthalsäure, Terephthalsäure, Benzoltricarbonsäure, Ölsäure und Ricinolsäure.

Unter einem "Polyamin" wird in dieser Anmeldung jede Verbindung verstanden, die wenigstens zwei Aminogruppen enthält. Besagtes Polyamin hat vorzugsweise ein Molekulargewicht von höchstens 300 g/mol. Bevorzugte Polyamine, die niedermolekulare Abbauprodukte der polyesterbasierten Polyurethanschäume sind, sind ausgewählt aus der Gruppe bestehend aus 4,4'-Methylendiamin, 2,4'-Methylendiamin, 2,2'-Methylendiamin, 2,4- Toluylendiamin, 2,6- Toluylendiamin, Hexamethylendiamin, Isophorondiamin, Xylylendiamin, Pentamethylendiamin, para-Phenylendiamin, Butyldiamin und H12-Methylendiamin. Besonders bevorzugt sind die Polyamine ausgewählt aus der Gruppe bestehend aus 4,4'-Methylendiamin, 2,4'-Methylendiamin, 2,2'-Methylendiamin, 2,4-Toluylendiamin und 2,6-Toluylendiamin.

Das erfindungsgemäße Verfahren ermöglicht ein effektives Recycling von Polyurethanen in zweierlei Hinsicht: (i) Das Verfahren selbst benötigt aufgrund der milden Reaktionsbedingungen keinen hohen Energieeinsatz und ermöglicht (ii) die stoffliche Verwertung des Polyurethans, da definierte Abbauprodukte entstehen, die ihrerseits wertvolle chemische Grundstoffe sind.

Im Vergleich dazu erfolgt die thermische Glykolyse, die zurzeit die gängigste Chemolyse zum Recycling von Polyurethan darstellt und bereits industriell umgesetzt ist, bei sehr hohen Temperaturen. Dabei liegt der Fokus auf der Gewinnung der Polyole, während die Amine als Störfaktor abgetrennt und nicht zurückgewonnen werden. Bei der nicht-enzymatischen Hydrolyse werden sowohl Polyole als auch Amine als Produkte gewonnen. Dieses Verfahren erfolgt jedoch bei hohen Temperaturen und hohen Umgebungsdrücken.

### Übersicht über die Abbildungen:

- Abbildung 1:: Ergebnis der phylogenetischen Analyse der in der vorliegenden Anmeldung offenbarten Aminosäuresequenzen

Die folgenden Ausführungsbeispiele dienen nur dazu, die Erfindung zu erläutern. Sie sollen den Schutzumfang der Patentansprüche in keiner Weise beschränken.

### Beispiele

### Test der Enzymaktivität mit ENPC

0,2 mg/ml ENPC wurden in 100 mM KH₂PO₄/K₂HPO₄ bei pH 7,0 mit 6,25 Vol.-% Ethanol bei Raumtemperatur und 900 rpm auf dem Plattenschüttler "MTS 2/4" (IKA, Staufen) für 24 Stunden inkubiert.

Nach dem Filtrieren der Proben wurden jeweils 100 µL in transparente 96-well-Platten mit flachem Boden "UV-Star" (Greiner Bio-One, Frickenhausen) übertragen und die Extinktion bei 405 und 480 nm bestimmt. Der Wert bei 480 nm wurde gemessen, da 4-Nitroanilin hier nicht mehr signifikant absorbiert und es somit bei hohen Werten für beide Wellenlängen sehr wahrscheinlich war, dass nicht 4-Nitroanilin sondern ein anderer Stoff für die Extinktion bei 405 nm verantwortlich war.

Bei der Hydrolyse durch Urethanasen wird das fast farblose ENPC zu 4-Nitroanilin, CO₂ und Ethanol gespalten, wobei 4-Nitroanilin bei 405 nm im Mikrotiterplatten-Photometer "Infinite M1000PRO" (Tecan, Männedorf, Schweiz) detektiert werden konnte. Zur Steuerung des Photometers wurde die Software "i-control" (Tecan, Männedorf, Schweiz) in der Version 3.4.2.0 verwendet. Zudem wurde 4-Nitroanilin mit der Methode "Dabsylamin" per HPLC detektiert.

### Hochdruckflüssigchromatographie (HPLC)

Die Hochdruckflüssigchromatographie wurde an einem Gerät der 1100er Serie von Agilent Technologies (Santa Clara, USA) mit *Autosampler* und DAD (*diode array detector*) für UV und den sichtbaren Bereich des Lichtes durchgeführt. Für alle Messungen wurde die Säule "Zorbax XDB-C18" mit einer Partikelgröße von 3,5 µm und den Abmessungen 4,6 x 75 mm (Agilent Technologies, Santa Clara, USA) verwendet. Bei allen Methoden wurden 5 µL Probe injiziert und die Säule auf 40 °C temperiert. Der Fluss lag generell bei 1,5 mL/min. Wegen der Verwendung einer Umkehrphasensäule wurde bei allen Methoden mit steigenden Konzentrationen an organischem Lösemittel eluiert.

Zur Detektion und Quantifizierung von dabsylierten aliphatischen Aminen und Urethanen wurde die Methode "Dabsylamin" verwendet. Aromatische Amine und Urethane konnten wegen der hohen Eigenabsorption ohne Derivatisierung mit dieser Methode quantifiziert werden. Als Laufmittel wurde neben AcN 10 mM Natriumphosphatpuffer pH 7,0 verwendet, dem zum Schutz vor mikrobiellem Wachstum 0,005% (w/v) Natriumazid zugegeben wurde. Um Druckproblemen durch verunreinigte Pumpenventile vorzubeugen wurde dem AcN später 5% (v/v) ddH₂O zugegeben und die Methode angepasst ("Dabsylamin95"). Zur Bestimmung der MDEC-Menge aus den enzymkatalysierten Reaktionen von 4,4'-MDA mit EC wurde die Methode "Dabsylamin-12-MeOH" verwendet, wobei die wässrige Komponente angesäuert ist und die dadurch protonierten aromatischen Amine sehr früh eluieren. Zur Untersuchung der Reaktionen von 4,4'-MDA mit DMC, 2,4-TDA mit DMC und 2,4-TDA mit EC wurde die Methode "Dabsylamin95-H2O" genutzt, welche sich von "Dabsylamin95" lediglich durch die Verwendung von reinem ddH₂O anstatt Puffer unterscheidet. Die Analyse der Daten erfolgte mit der Software "OpenLAB CDS ChemStationLC" in der Version A.02.09[017] (Agilent Technologies, Santa Clara, USA).

Dabsylamin: Laufmittel: AcN und 10 mM Na₂HPO₄/NaH₂PO₄, pH 7,0

| t [min] | AcN |
|---|---|
| 0 | 5 |
| 6,5 | 85 |
| 8,0 | 5 |
| 10,0 | 5 |

Dabsylamin95: Laufmittel: AcN mit 5% (v/v) ddH₂O und 10 mM Na₂HPO₄/NaH₂PO₄, pH 7,0

| t [min] | % AcN (+5% (v/v) ddH₂O) |
|---|---|
| 0 | 5 |
| 6,5 | 90 |
| 8,0 | 5 |
| 10,0 | 5 |

Dabsylamin-12-MeOH-lang: Laufmittel: Methanol und ddH₂O mit 0,1% (v/v) Methansäure

| t [min] | % Methanol |
|---|---|
| 0 | 5 |
| 2,5 | 35 |
| 8,0 | 70 |
| 8,5 | 85 |
| 10,0 | 5 |
| 12,0 | 5 |

| | | |
|---|---|---|
| **SEQ ID Nr.** | **Bezeichnung in Studie** | **Hydrolyse von ENPC** |
| 1 | GatA61 | + |
| 2 | Aes70 | + |
| 3 | Aes72 | + |
| 4 | Aes170 | + |
| 5 | Aes174 | + |
| 6 | Aes175 | + |
| 7 | GatA197 | + |
| 8 | Aes214 | + |
| 9 | GatA250 | + |
| 10 | AesGö56 | + |
| 11 | SB12 | + |
| 12 | SB23 | + |

### Test der Enzymaktivität mit EPEC

Der Versuch wurde wie für ENPC beschrieben durchgeführt. Das entstehende Phenethylamin wurde durch HPLC nachgewiesen wie oben beschrieben.

| **SEQ ID Nr.** | **Bezeichnung in Studie** | **Hydrolyse von EPEC** |
|---|---|---|
| 1 | GatA61 | + |
| 2 | Aes70 | - |
| 3 | Aes72 | + |
| 4 | Aes170 | - |
| 5 | Aes174 | + |
| 6 | Aes175 | - |
| 7 | GatA197 | + |
| 8 | Aes214 | + |
| 9 | GatA250 | + |
| 10 | AesGö56 | - |
| 11 | SB12 | + |
| 12 | SB23 | + |

### Phylogenetische Analyse der Enzyme

Zur Erstellung von Stammbäumen zur Verwandtschaft der Urethanasen wurde das Programm "MegAlign" (DNASTAR, Madison, USA) in der Version 10.1.0 verwendet. Stammbäume wurden mit den Standardeinstellungen unter Nutzung von "ClustalW" erstellt.

Für die Erstellung von *Alignments* verschiedener Proteine wurde das Programm "Clustal Omega" verwendet (Sievers *et al.,* 2011).

Die Datenbanksuche nach Proteinsequenzen wurde mit BLASTP durchgeführt (Altschul *et al.,* 1990).

*Open Reading Frames* (ORFs) in sequenzierten Metagenomsequenzen wurden mit dem Onlineprogramm "ORF-Finder" von NCBI (Wheeler *et al.,* 2007) ausfindig gemacht.

Identische Hydrolasegene wurden auf einen Vertreter reduziert und alle Sequenzen mit ORFs untersucht, um die kompletten Sequenzen der Gene zu erhalten. Bei der Suche wurden auch alternative Startcodons zugelassen. Hierbei fiel auf, dass für das Gen aus pLip214 ein *N*-terminaler Bereich mit Ähnlichkeit zu *aes* vorhanden war, wobei kein Startcodon gefunden werden konnte. Dieser Genabschnitt lag nicht am Rand des Inserts des Metagenomvektors, was ein verkürztes Gen erklären könnte. Für weitere Analysen wurde als Sequenz für dieses Gen der Bereich mit Ähnlichkeit zu *aes* ohne Startcodon genutzt. Die identifizierten putativen Urethanasegene wurden *in silico* translatiert und über BLASTP mit der NCBI-Datenbank abgeglichen. Die putativen Urethanasen wurden gemäß ihrer Nummer in der Lipasebank und der Ähnlichkeit zu GatA oder Aes benannt.

Um die einzelnen Mitglieder der beiden identifizierten Urethanasegruppen (GatA und Aes) untereinander zu vergleichen wurde jeweils ein *Alignment* mit dem Programm "Clustal Omega" gebildet und zusätzlich ein Stammbaum mit der Programm "MegAlign" erstellt, wobei für den Stammbaum ein geneimsames *Alignment* beider Gruppen erstellt wurde. Es wurden auch die Sequenzen für die Enzyme aus der Literatur (Ure, Ana und NfpolyA), die alle Ähnlichkeit mit GatA aufweisen, mit in den Sequenzvergleich einbezogen.

Der Stammbaum ist in Abbildung 1 dargestellt. Es zeigt sich, dass beide Gruppen in unterschiedlichen Ästen lokalisiert sind, wobei die Ähnlichkeitsverhältnisse innerhalb der beiden Gruppen teilweise nicht so deutlich waren, was an geringeren *Bootstrapping-Werten* an den Knotenpunkten zu erkennen ist. Innerhalb der GatA-Gruppe scheinen größere Unterschiede zu existieren als innerhalb der Aes-Gruppe, was an den größeren Astlängen in dieser Gruppe zu erkennen ist. Vor allem Aes70 und Aes72 sowie Aes175 und Aes214 weisen eine sehr hohe Ähnlichkeit auf, was sowohl durch die relativ kurzen Äste im Stammbaum als auch durch das Auffinden des gleichen Proteins mit der größten Ähnlichkeit bei der BLASTP-Suche äußerte.

### Herstellung des Polyurethanschaums für die Abbauversuche

In für die Herstellung von Polyurethanschaumstoffen üblicher Verarbeitungsweise nach dem Einstufenverfahren wurden die unten aufgeführten Einsatzstoffe miteinander zur Reaktion gebracht. Die Rohdichte betrug 38 kg/m3 (DIN EN ISO 845 in der Fassung vom Oktober 2009), die Stauchhärte bei 40% Stauchung 3,5 kPa (DIN EN ISO 3386-1 in der Fassung vom Oktober 2015)

### Rezeptur:

| | |
|---|---|
| 100 Teile | Desmophen 2200B |
| 3 Teile | Wasser |
| 19 Teile | Desmodur T80 |
| 19 Teile | Desmodur T65 |
| 0,7 Teile | N,N'-Dimethylpiperazin |
| 1 Teil | Tegostab 8325 |

### Rohstoffe:

Desmophen^{®}2200B, Fa. Covestro Deutschland AG; verzweigtes Polyesterpolyol auf Basis Adipinsäure, Diethylenglykol und 1,1,1-Trimethylolpropan mit einer Hydroxylzahl von ca. 60 mg KOH/g.

Desmodur^{®}T80, Fa. Covestro Deutschland AG; Isomerengemisch aus 2,4- und 2,6-Toluylendiisocyanat mit einem Mischungsverhältnis von ca. 80:20.

Desmodur^{®}T65, Fa. Covestro Deutschland AG; Isomerengemisch aus 2,4- und 2,6-Toluylendiisocyanat mit einem Mischungsverhältnis von ca. 67: 33.
N,N'-Dimethylpiperazin, Katalysator der Fa. abcr GmbH
Tegostab^{®}B 8325, Schaumstabilisator der Fa. Evonik
Wasser; entionisiertes Wasser
Die Rezeptur kann mit Kennzahlen von 90 bis 115 ausgeführt werden. Mit Kennzahl wird das mit 100 multiplizierte molare Verhältnis von Isocyanatgruppen zu isocyanatreaktiven Gruppen bezeichnet.

### Abbau von Polyurethanschaum

Als Substrat wurde ein mit Toluylendiisocyanat hergestelltes Polyesterpolyurethan verwendet. Der Abbau erfolgte in zwei Reaktionsschritten. Zunächst wurde der Schaum mit einer Lipase inkubiert. Die hierbei entstehenden Oligomere wurden nach Neutralisation mit einer Urethanase in Monomere aufgespalten.

Im ersten Schritt wurden 1 g des Schaumes mit 20 mL Kaliumphosphatpuffer pH 7,0 und ca. 30 mg CalB-Lyophilisat "Chirazyme L2" (Roche, Basel, Schweiz) (hier als SEQ ID Nr. 12 bezeichnet) in ein 50-ml Zentrifugenröhrchen gegeben und bei 37 °C und 200 rpm für 5 Tage inkubiert. Stücke der Schaumreste wurden im Vergleich zu einer Negativkontrolle ohne Enzym mit dem Mikroskop "MH2" (Olympus, Hamburg) fotografiert. Anschließend wurde die trübe Lösung bei 25 °C und 4000 rpm in einer Großraumzentrifuge für 10 Minuten zentrifugiert. Der klare Überstand wurde mit 1 M NaOH auf pH 7,0 eingestellt. Nach ca. 6 Stunden bei Raumtemperatur wurde der leicht gefallene pH-Wert erneut auf 7,0 titriert und die Lösung sterilfiltriert. Die löslichen Oligomere wurden bis zur Verwendung bei 4 °C gelagert.

Zur weiteren Verwendung wurden die löslichen Oligomere in 1,5-mL-Reaktionsgefäße gegeben, mit 20 µL DMF und 150 µL des für die jeweilige Urethanase optimalen Puffers (100 mM Natriumphosphatpuffer, eingestellt auf das jeweilige Optimum der Urethanse im Bereich pH 6,0 bis pH 8,0) versetzt. Anschließend wurden jeweils 30 µL der unverdünnten, aufgereinigten Urethanasen zugegeben und die Ansätze bei 30 °C und 1000 rpm auf dem Thermoblock geschüttelt. Als Negativkontrolle diente ein Ansatz mit Enzymlagerpuffer. Nach drei Tagen wurden die Ansätze über Filterplatten mit PVDF-Membran und 0,2 µm Porengröße (Corning, Kaiserslautern) gefiltert und das Filtrat per HPLC mit der Methode "Dabsylamin95" auf entstandenes 2,4- und 2,6-Toluylendiamin hin untersucht.

Bereits nach der Reaktion im Ansatz mit dem CalB-Lyophilisat war makroskopisch im Vergleich zu einer Negativkontrolle ohne Enzym zu erkennbar, dass der Schaum jegliche Struktur verloren hatte und als trübe Suspension mit kleinen Schaumpartikeln vorlag. Der zu Anfang des Experimentes fast vollständig vom Schaum aufgenommene Puffer beinhaltete später die gesamte Schaummasse als zersetzte Partikel. Bei der HPLC-Analyse zeigten sich deutliche Peaks, die den gebildeten Oligomeren zugeschrieben wurden, aber keine Peaks, die auf die Bildung von Toluylendiamin (TDA) hindeuten würden (Daten nicht gezeigt).

Die Oligomerlösung wurde mit allen exprimierten Urethanasen und SEQ ID Nr. 12 versetzt und anschließend per HPLC auf die Bildung von TDA untersucht. Dieses konnte für die Ansätze mit SEQ ID Nr. 7 und SEQ ID Nr. 3 nachgewiesen werden, wobei die gemessene Menge von 2,6-TDA in beiden Ansätzen etwa gleich war und die Bildung von 2,4-TDA im Ansatz mit Enz03 deutlich stärker ausfiel. Mit SEQ ID Nr. 3 wurden 0,057 g/L 2,4-TDA und 0,025 g/L 2,6-TDA gebildet, während SEQ ID Nr. 7 zur Bildung von 0,0075 g/L 2,4-TDA und 0,024 g/L 2,6-TDA führte. Weiterhin zeigte sich mit diesen beiden Enzymen im Gegensatz zu den anderen Ansätzen eine Veränderung und generelle Verkleinerung der Oligomerpeaks. Im Falle von SEQ ID Nr. 7 konnte bereits TDA ohne vorherige Vorbehandlung aus dem Polyester-PU-Schaum abgespalten werden, während dies im Falle von SEQ ID Nr. 3 erst durch die Bereitstellung neutralisierten von Oligomeren nach vorheriger Esterspaltung möglich war. Die Tatsache, dass die als Oligomerpeaks identifizierten Produktpeaks aus der Hydrolyse mit SEQ ID Nr. 12 nach weiterer Behandlung mit Urethanasen drastisch verkleinert wurden, wobei eine deutliche TDA-Bildung auftrat, bestätigte, dass es sich bei diesen Peaks um Oligomere handelte.

Es konnte also gezeigt werden, dass unlöslicher auf TDI-basierender aus Polyester-Polyurethan Schaum durch eine Kombination von zwei Reaktionsschritten in seine Monomere aufgespalten werden kann. In einem ersten Schritt wurde unter Einsatz der Lipase CalB über die Hydrolyse der Esterbindungen der PU Schaum vorverdaut. Nach Neutralisation wurden die frei gewordenen Oligomere als Substrat für die überexprimierten Urethanasen eingesetzt. Dabei wurden die Urethanbindungen hydrolysiert und TDA konnte als Monomer nachgewiesen werden.

## Patentansprüche

1. Polypeptid mit einer Aminosäuresequenzgemäß SEQ ID Nr. 4 oder eine Variante davon, **dadurch gekennzeichnet dass** das Polypeptid Urethanaseaktivität aufweist.

2. Polypeptid gemäß Anspruch 1, wobei die Variante durch die Hinzufügung, Deletion oder Austausch von bis zu 10 % der im jeweiligen Polypeptid enthaltenen Aminosäuren erhalten wird.

3. Verwendung eines Polypeptids mit einer Aminosäuresequenz gemäß SEQ ID Nr. 4 oder einer Variante dieses Polypeptids, **dadurch gekennzeichnet, dass** das Polypeptid Urethanaseaktivität aufweist, zur enzymatischen Spaltung von Urethanbindungen.

4. Verwendung gemäß Anspruch 3, wobei die Urethangruppe aromatisch gebunden ist.

5. Verfahren zum Abbau von Polyester-Polyurethanen in niedermolekulare Abbauprodukte enthaltend die Schritte
a) Spaltung der im Polyester-Polyurethan enthaltenen Estergruppen; und
b) Behandlung des Polyurethans mit einem Polypeptid, welches Urethanaseaktivität aufweist und eine Aminosäuresequenz hat wie in SEQ ID NO. 4 definiert oder wenigstens 90 % Sequenzidentität zu der vorgenannten Sequenz hat;
mit der Maßgabe, dass die Verfahrensschritte a) und b) in beliebiger Reihenfolge oder auch gleichzeitig durchgeführt werden können.

6. Das Verfahren nach Anspruch 5, wobei der Verfahrensschritt a) vor dem Verfahrensschritt b) durchgeführt wird.

7. Das Verfahren nach einem der Ansprüche 5 oder 6, wobei als Verfahrensprodukte Polyole, Polycarbonsäuren und Polyamine entstehen.

8. Das Verfahren nach Anspruch 7, wobei wenigstens ein Polyamin ausgewählt aus der Gruppe bestehend aus 4,4'-Methylendiamin, 2,4'-Methylendiamin, 2,2'-Methylendiamin, 2,4-Toluylendiamin und 2,6-Toluylendiamin entsteht.

9. Das Verfahren nach Anspruch 7, wobei wenigstens ein Polyol ausgewählt aus der Gruppe bestehend aus Ethylengylcol, Diethylenglykol, Triethylenglycol, Propylenglycol, 1,2- Di-Propylenglycol, Neopentylglykol, Glycerin, 1,1,1-Trimethylolpropan, Saccharose, Sorbitol und Pentaerythrit entsteht.

10. Das Verfahren nach Anspruch 7, wobei wenigstens eine Polycarbonsäure ausgewählt aus der Gruppe bestehend aus Bernsteinsäure, Glutarsäure, Adipinsäure, Phthalsäure, Terephthalsäure, Benzoltricarbonsäure, Ölsäure und Ricinolsäure entsteht.

11. Das Verfahren nach einem der Ansprüche 5 bis 10, wobei der Verfahrensschritt a) mit einer Lipase durchgeführt wird.

12. Das Verfahren nach Anspruch 11, wobei die Lipase eine Aminosäuresequenz wie in SEQ ID Nr. 11, SEQ ID Nr. 12 oder einer Variante der vorgenannten Sequenzen mit wenigstens 90 % Sequenzidentität zu SEQ ID Nr. 11 oder SEQ ID Nr. 12 hat.
